(19) Europäisches Patentamt
European Patent Office
Office européen des brevets

(11) Publication number: 0 245 619 B1

(12) **EUROPEAN PATENT SPECIFICATION**

(45) Date of publication of the patent specification: **11.07.90**

(51) Int. Cl.[5]: **C07D 417/12**

(21) Application number: **87103997.0**

(22) Date of filing: **05.08.83**

(60) Publication number of the earlier application in accordance with Art. 76 EPC: **0101265**

# (54) Novel thiazolylacetic acid derivatives and processes for preparing the same.

(30) Priority: **07.08.82 GB 8222823**

(43) Date of publication of application: **19.11.87 Bulletin 87/47**

(45) Publication of the grant of the patent: **11.07.90 Bulletin 90/28**

(84) Designated Contracting States: **AT BE CH DE FR GB IT LI LU NL SE**

(56) References cited: **None**

(73) Proprietor: **Tanabe Seiyaku Co., Ltd., No. 21 Dosho-machi 3-chome Higashi-ku, Osaka-shi Osaka-fu(JP)**

(72) Inventor: **Oine, Toyonari, 14-4 Higashi-Tomigaoka, 2-chome, Nara-shi Nara-ken(JP)**
Inventor: **Sugano, Hiroshi, A6 110 4162-53, Naka-Tomigaoka, 1-chome, Nara-shi Nara-ken(JP)**
Inventor: **Yamada, Yoshihisa, 10 Naeshiromoto-machi Zushioku Yamashina-ku, Kyoto-shi Kyoto-fu(JP)**
Inventor: **Yamaguchi, Totaro, 4-7 Ryoke 7-chome, Urawa-shi Saitama-ken(JP)**
Inventor: **Ohshima, Satoshi, 4073 Oaza-Iwatsuki, Iwatsuki-shi Saitama-ken(JP)**

(74) Representative: **Taylor, Phillip Kenneth et al, W.P. THOMPSON & CO. Coopers Building Church Street, Liverpool L1 3AB(GB)**

EP 0 245 619 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel thiazolylacetic acid derivatives and processes for preparing the same. More particularly, it relates to a compound of the formula:

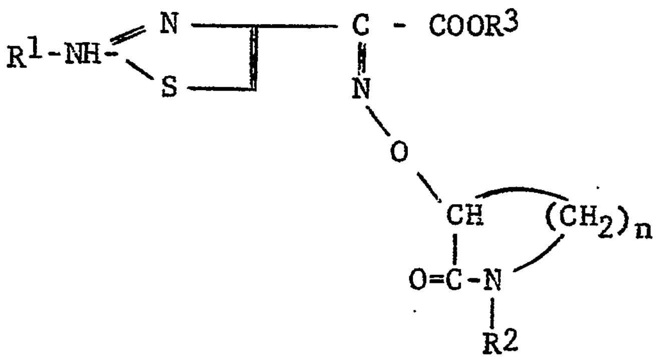

(I)

wherein $R^1$ is a protective group usually employed to protect amino groups in peptide synthesis, $R^2$ is H or an alkyl radical having 1 to 4 carbon atoms, $R^3$ is H or $C_2H_5$ and n is an integer of 2 or 3.

The new thiazolylacetic acid derivatives of the present invention are useful as intermediates in the synthesis of cephalosporins.

Among the compounds of the present invention, preferred groups when $R^2$ is lower alkyl are methyl, ethyl and propyl.

A wide variety of protecting groups which have usually been employed to protect an amino group in the peptide synthesis can be used as the protecting group $R^1$. Examples of such protecting groups are lower alkanoyl, e.g. formyl, acetyl and pivaloyl; mono, di- or trihalogeno-lower alkanoyl, e.g. chloroacetyl and trifluoroacetyl; lower alkoxycarbonyl, e.g. methoxycarbonyl, ethoxycarbonyl and tert.-butoxycarbonyl; substituted or unsubstituted benzyloxycarbonyl, e.g. benzyloxycarbonyl and p-methoxybenzyloxycarbonyl; substituted or unsubstituted phenyl-lower alkyl, e.g. p-methoxybenzyl and 3,4-dimethoxybenzyl; and di- or triphenyl lower alkyl, e.g. benzhydryl and trityl. Moreover, while the compound (I) can exist in the form of two optical isomers due to the asymmetric carbon atom involved in the group of the formula:

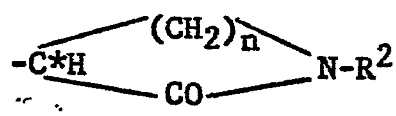

(wherein the asterisk denotes an asymmetric carbon atom), either an optical isomer of the compound (I) or a racemic modification thereof may be used for the purpose of the present invention. Throughout the specification and claims, "levorotatory isomer" of the compound (I) in which $R^2$ is hydrogen and n is an integer of 2 means that the absolute configuration of said compound at said asymmetric carbon atoms is S-configuration and also "dextrorotatory isomer" means that the absolute configuration of said compound at said asymmetric carbon atom is R-configuration.

The compounds (I) of the present invention may be prepared, for example, by reacting a compound of the formula

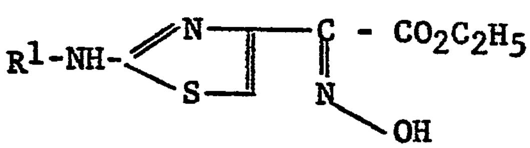

(II)

wherein $R^1$ is the same as defined above, with a compound of the formula:

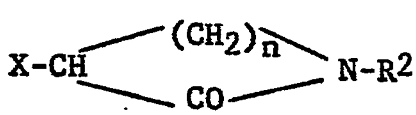

(III)

(wherein X is a halogen and $R^2$ and n are the same as defined above) in the presence of an alkali (e.g., potassium carbonate) in a solvent (e.g., dimethyl-sulphoxide) at a temperature of 10 to 50°C to give a

compound of the formula:

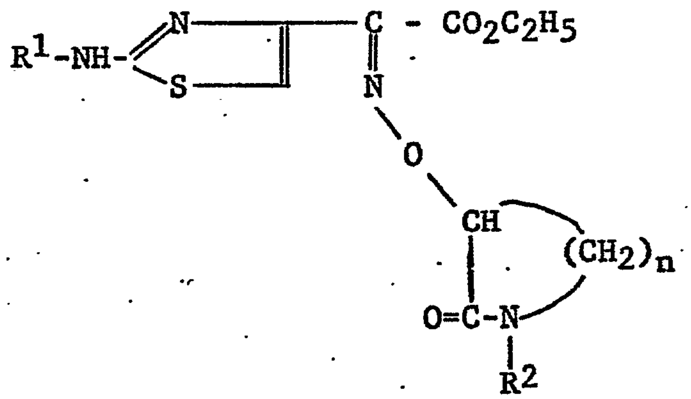

(IV)

wherein $R^1$, $R^2$ and n are the same as defined above, and if desired hydrolyzing the compound (IV). Alternatively, the compound (I) in which $R^3$ is hydrogen may be prepared by hydrolyzing the compound (II) to give a compound of the formula:

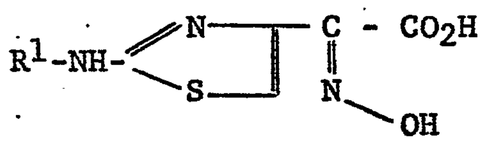

(V).

wherein $R^1$ is the same as defined above, and then reacting the compound (V) with a compound of the formula:

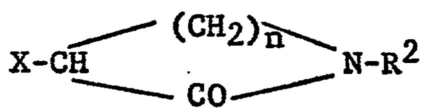

(III)

(wherein $R^2$, n and X are the same as defined above) in the presence of an acid acceptor (e.g., sodium hydride) at a temperature of 10 to 40°C in a solvent (e.g., dimethyl sulphoxide). Moreover, as mentioned hereinbefore, the compound (I) involves two optical isomers due to the asymmetric carbon involved in the group of the formula:

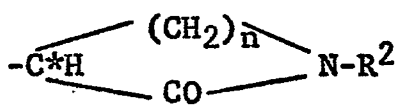

(wherein the asterisk denotes an asymmetric carbon atom). If required, however, such optical isomers may be separated into each optical isomer by optical resolution thereof. For example, the compound (I) in which $R^2$ and $R^3$ are hydrogen, n is an integer of 2 and $R^1$ is trityl can be readily separated into each optical isomer by reacting the racemic modification of the compound (I) with L- and D-phenylalanine methyl ester in a solvent (e.g., a mixture of methanol and dioxane) to form the diastereoisomeric salts thereof, and separating said diastereoisomers into each component thereof by selective recrystallization. By said selective recrystallization, the least soluble diastereoisomer is recovered as crystals from the reaction mixture and the more soluble diastereoisomer remains soluble therein. It is preferred to carry out said selective crystallization at a temperature of 10 to 40°C.

Practical and presently-preferred embodiments of the present invention are illustratively shown in the following Examples. Throughout the specification and claims, the term "lower alkyl" should be interpreted as referring to alkyl having one to four carbon atoms.

Example (1)

15.8g of ethyl (Z)-2-(2-tritylaminothiazol-4-yl)-2-hydroxyiminoacetate are dissolved in 70ml of dimethylsulfoxide, and 5.8g of anhydrous potassium carbonate are added thereto. The mixture is stirred at room temperature for 20 minutes. 6.6g of 3-bromo-2-pyrrolidone are added to said mixture, and the mixture is stirred at room temperature for 20 hours. The mixture is poured into 800 ml of water, and crystalline precipitates are collected by filtration and washed with water. The crystals are dissolved in chloroform, washed with water and then dried. Then, the chloroform solution is evaporated under reduced pressure to remove solvent. 100ml of ethyl acetate are added to the residue, and allowed to stand at room temperature. Crystalline precipitates thus obtained are collected by filtration and dried. 16.0g of ethyl (Z)-2-(2-tritylaminothiazol-4-yl)-2-[(2-pyrrolidon-3-yl)oxyimino]acetate are obtained.
M.p. 209 - 210°C
NMR ($CDCl_3$)δ : 1.30 (3H, t, J=7Hz), 2.1-2.6 (2H, m) 3.1-3.6 (2H, m), 4.34 (2H, q, J=7Hz) 4.90 (1H, t, J=7Hz), 6.53 (1H, s) 7.0-7.6 (17H, m)

16.0g of ethyl (Z)-2-(2-tritylaminothiazol-4-yl)-2-[(2-pyrrolidon-3-yl)oxyimino]acetate are added to a mixture of 160ml of methanol and 30ml of an aqueous 2N sodium hydroxide solution, and the mixture is refluxed for 30 minutes under heating. After cooling, crystalline precipitates are collected by filtration and washed with methanol. The crystals are suspended in 30ml of water. Then, the suspension is adjusted to pH 3 with 2N hydrochloric acid. Crystalline precipitates are collected by filtration and dried. 11.4g of (Z)-2-(2-tritylaminothiazol-4-yl)-2-[(2-pyrrolidon-3-yl) oxyimino]acetic acid are obtained.
M.p. 150 - 153°C (decomp.)
NMR (DMSO-$d_6$)δ : 1.8-2.4 (2H, m), 2.9-3.4 (2H, m) 4.63 (1H, t, J=7Hz), 6.76 (1H, s) 6.9-7.6 (15H, m), 7.85 (1H, s) 8.70 (1H, broad s)

Example (2)

30g of (Z)-2-(2-tritylaminothiazol-4-yl)-2-[(2-pyrrolidon-3-yl)oxyimino]acetic acid and 60ml of methanol are added to 100ml of dioxane containing 10.5g of methyl L-phenylalaninate, and the mixture is heated at 50°C to dissolve said acid therein. 700ml of dioxane are added to the solution, and the mixture is stirred at room temperature for 5 hours. Crystalline precipitates are collected by filtration (the filtrate is hereinafter referred to as "Filtrate I"), and 14.3g of the crude product thus obtained are dissolved in 24ml of methanol. 280ml of dioxane are added to the methanol solution. The mixture is stirred at room temperature for 4 hours, and crystalline precipitates are collected by filtration (the filtrate is hereinafter referred to as "Filtrate II"). 12.2g of (Z)-2-(2-tritylaminothiazol-4-yl)-2-[(2-pyrrolidon-3-yl)oxyimino]acetic acid (ℓ-isomer) methyl L-phenylalaninate salt are obtained.

$$[\alpha]_D^{25} - 14.0^\circ \text{ (C=1, methanol)}.$$

12.2g of the above-mentioned salt are dissolved in 120ml of methanol, and 176ml of 0.1N hydrochloric acid are added thereto. The mixture is stirred for 2 hours under ice-cooling. Crystalline precipitates are collected by filtration and washed with methanol. 7.5g of (Z)-2-(2-tritylaminothiazol-4-yl)-2-[(2-pyrrolidon-3-yl)oxyimino]acetic acid (ℓ-isomer) are obtained. Another designation of this levorotatory isomer is shown as (Z)-2-(2-tritylaminothiazol-4-yl)-2-[((3S)-2-pyrrolidon-3-yl)oxyimino]acetic acid.
M.p. 142-143°C (decomp.)

$$[\alpha]_D^{25} - 38.8^\circ \text{ (C=1, dimethylformamide)}$$

Example (3)

Filtrate I and II obtained in the above-mentioned Example (2) are condensed to dryness under reduced pressure. The residue is dissolved in 250ml of methanol and then 450ml of 0.1N hydrochloric acid are added dropwise to the solution. The mixture is stirred for 2 hours under ice-cooling. The resulting crystalline precipitates are collected by filtration, washed with methanol, and dried. 20g of (Z)-2-(2-tritylaminothiazol-4-yl)-2-[(2-pyrrolidon-3-yl)-oxyimino]acetic acid (containing excess of the d-isomer) are obtained. 20.0g of (Z)-2-(2-tritylaminothiazol-4-yl)-2-[(2-pyrrolidon-3-yl)-oxyimino]acetic acid thus recovered and 40ml of methanol are added to 70 ml of dioxane containing 7.0g of methyl D-phenylalaninate, and the mixture is heated at 50°C to dissolve said acid therein. 450ml of dioxane are added to said solution. Then, the mixture is stirred at room temperature for 4 hours, and crystalline precipitates are collected by filtration. 13.3g of the crude product thus obtained are dissolved in 20ml of methanol, and 260ml of dioxane are added thereto. The mixture is stirred at room temperature for 4 hours. Crystalline precipitates are collected by filtration. 12.0g of (Z)-2-(2-tritylaminothiazol-4-yl)-2-[(2-pyrrolidon-3-yl)oxyimino]acetic acid (d-isomer) methyl D-phenylalaninate salt are obtained.

$$[\alpha]_D^{25} + 13.9° \text{ (C=1, methanol)}$$

12.0g of the above-mentioned salt are dissolved in 120ml of methanol, and 174ml of 0.1N hydrochloric acid are added thereto. The mixture is stirred for 2 hours under ice-cooling. Crystalline precipitates are collected by filtration and washed with methanol. 7.3g of (Z)-2-(2-tritylaminothiazol-4-yl)-2-[(2-pyrrolidon-3-yl)oxyimino]acetic acid (d-isomer) are obtained. Another designation of this dextrorotory isomer is shown as (Z)-2-(2-tritylaminothiazol-4-yl)-2-[((3R)-2-pyrrolidon-3-yl)oxyimino]acetic acid.
M.P. 143–144°C (decomp.)

$$[\alpha]_D^{25} + 37.4° \text{ (C=1, dimethylformamide)}$$

Example (4)

2.7g of ethyl (Z)-2-(2-tritylaminothiazol-4-yl)-2-hydroxyiminoacetate are dissolved in 12ml of dimethylsulfoxide, and 1.0g of anhydrous potassium carbonate is added thereto under nitrogen gas atmosphere. The mixture is stirred at room temperature for 10 minutes. 1.2g of 1-methyl-3-bromo-2-pyrrolidone are added to the mixture, and the mixture is stirred at room temperature for 5 hours. The reaction mixture is poured into 100ml of water, and crystalline precipitates are collected by filtration. The crystals are dissolved in ethyl acetate, and the solution is washed with water and then dried. The solution is concentrated under reduced pressure to remove solvent. Then, the residue is crystallized with isopropyl ether and collected by filtration. 2.1g of ethyl (Z)-2-(2-tritylaminothiazol-4-yl)-2-[(1-methyl-2-pyrrolidon-3-yl)oxyimino]acetate are obtained.
NMR ($CDCl_3$) δ : 1.30 (3H, t, J=7Hz), 2.0-2.7 (2H, m) 2.88 (3H, s), 3.0-3.6 (2H, m) 4.34 (2H, q, J=7Hz), 4.92 (1H, t, J=7Hz), 6.54 (1H, s), 6.87 (1H, s) 7.0-7.5 (15H, m)

2.7g of ethyl (Z)-2-(2-tritylaminothiazol-4-yl)-2-[(1-methyl-2-pyrrolidon-3-yl)oxyimino]acetate are suspended in 27ml of methanol, and 4.9ml of 2N sodium hydroxide solution are added thereto. The mixture is refluxed for 20 minutes under heating. After cooling, the mixture is concentrated under reduced pressure to remove methanol. The residue is adjusted to pH 3 with 2N hydrochloric acid and extracted with ethyl acetate. The extract is dried and evaporated under reduced pressure to remove solvent. Then, the residue thus obtained is crystallized with ether and collected by filtration. 2.15g of (Z)-2-(2-tritylaminothiazol-4-yl)-2-[(1-methyl-2-pyrrolidon-3-yl)oxyimino]acetic acid are obtained.
M.p. 142 - 145° (decomp.)
NMR (DMSO-$d_6$)δ:2.0-2.5 (2H, m), 2.77 (3H, s) 3.1-3.4 (2H, m), 4.78 (1H, t, J=8Hz) 6.87 (1H, s), 6.9-7.5 (16H, m)

Example (5)

1.3g of (Z)-2-(2-tritylaminothiazol-4-yl)-2-hydroxyiminoacetic acid are dissolved in 10ml of dimethylformamide, and 0.24g of sodium hydride (60% oil dispersion) is added thereto. The mixture is stirred at room temperature for 15 minutes. 0.65g of 3-bromo-2-piperidone is added to the mixture, and the mixture is stirred at room temperature for 1.5 hours. The reaction mixture is poured into water and washed with a mixture of ethyl acetate and tetrahydrofuran (1:1). The aqueous layer is adjusted to pH 3 with 10% hydrochloric acid and extracted with a mixture of ethyl acetate and tetrahydrofuran (1:1). The extract is dried and concentrated to dryness under reduced pressure. Then, ether is added to the residue, and the resulting powder is collected by filtration. The powder (1.3g) is purified by silica gel chromatography (solvent, methanol:chloroform = 1:4). 0.85g of (Z)-2-(2-tritylaminothiazol-4-yl)-2-[(2-piperidon-3-yl)-oxyimino]acetic acid are obtained.
M.p. 145 - 150°C (decomp.)

## Claims

1. A compound characterised by the formula:

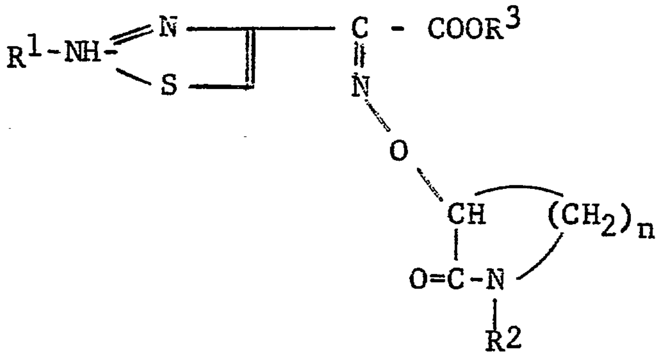

(I)

wherein $R^1$ is a protective group usually employed to protect amino groups in peptide synthesis, $R^2$ is H or an alkyl radical having 1 to 4 carbon atoms, $R^3$ is H or $C_2H_5$ and n is an integer of 2 or 3.

2. A compound as claimed in claim 1 in which $R^1$ is a trityl group.

3. A compound as claimed in claim 1 which is (Z)-2-(2-tritylaminothiazol-4-yl)-2-[(2-pyrrolidon-3-yl)oxyimino]acetic acid.

4. A compound as claimed in claim 1 which is ethyl (Z)-2-(2-tritylaminothiazol-4-yl)-2-[(2-pyrrolidon-3-yl) oxyimino]acetate.

5. A compound as claimed in claim 1 which is (Z)-2-(2-tritylaminothiazol-4-yl)-2-[(1-methyl-2-pyrrolidon3-yl)oxyimino]acetic acid.

6. A compound as claimed in claim 1 which is ethyl (Z)-2-(2-tritylaminothiazol-4-yl)-2-[(1-methyl-2-pyrrolidon-3-yl)oxyimino]acetate.

7. A compound as claimed in claim 1 which is (Z)-2-(2-tritylaminothiazol-4-yl)-2-[(2-piperidonyl)oxyimino]acetic acid.

8. A compound as claimed in claim 1 which is (Z)-2-(2-tritylaminothiazol-4-yl)-2-[((3S-2-pyrrolidon3-yl)-oxyimino]acetic acid.

9. A compound as claimed in claim 1 which is (Z) -2-(2-tritylaminothiazol)-4-yl)-2-[((3R)-2-pyrrolidon3-yl)-oxyimino]acetic acid.

10. A process for preparing a compound of the formula given in claim 1 which comprises reacting a compound of the formula:

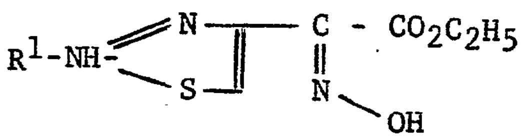

(II)

wherein $R^1$ has the meaning defined in claim 1, with a compound of the formula:

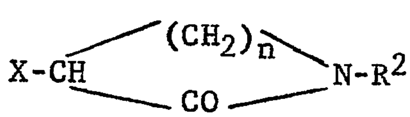

(III)

wherein X is halogen and $R^2$ and n are the same as defined in claim 1 to form a compound of the formula:

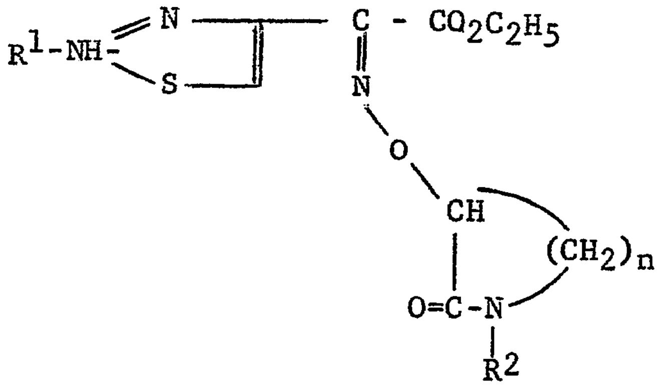

(IV)

wherein $R^1$ and $R^2$ have the meaning defined in claim 1 and if desired hydrolyzing the compound of formula IV.

11. A process for preparing a compound of the formula:

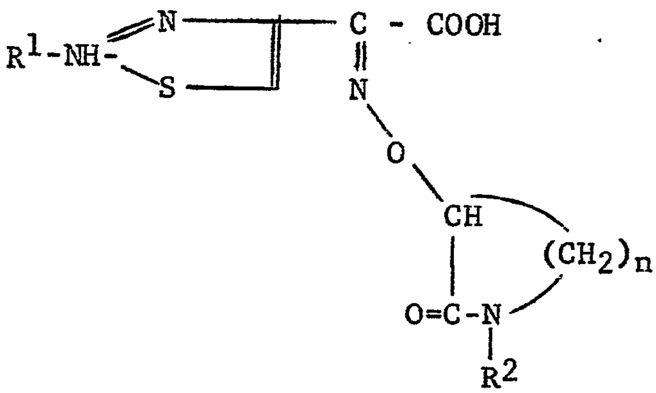

(VI)

wherein $R^1$, $R^2$ and n have the meanings defined in claim 1, which comprises hydrolyzing a compound of the formula:

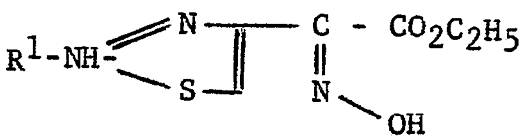

(II)

wherein $R^1$ is as defined in claim 1 to give a compound of the formula:

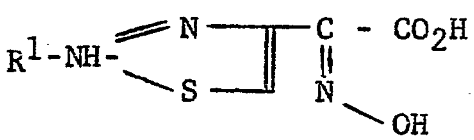

(V)

wherein $R^1$ has the meaning defined in claim 1 and then reacting the compound V with a compound of the formula:

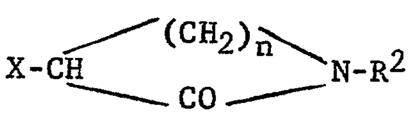

(III)

12. A process for preparing an optically active compound of the formula:

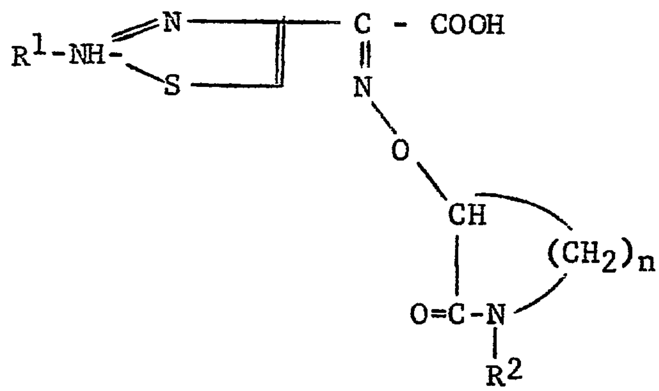

(VI)

wherein $R^1$ and $R^2$ and n have the meanings defined in claim 1, characterised by the steps of reacting a racemic modification of a compound of formula VI with an optically active resolving agent to form two diastereoisomeric salts thereof, separating said diastereoisomeric salts from each other.

13. A process as claimed in claim 12 characterised in that the optically active resolving agent is an L- or D-phenylalanine methyl ester and the separation of the diastereoisomers is effected by selective crystallization.

14. A process as claimed in claim 13 characterised in that the selective crystallisation is effected at a temperature of from 10° to 40°C.

**Revendications**

1. Un composé caractérisé par la formule:

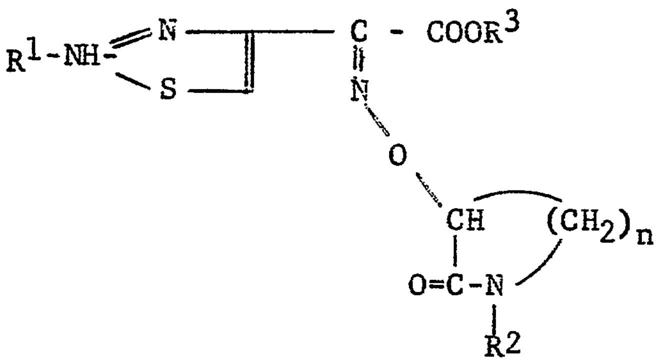

(I)

dans laquelle $R^1$ est groupe protecteur habituellement utilisé pour protéger les groupes amino dans la synthèse peptidique, $R^2$ est H ou un radical alkyle en $C_1$–$C_4$, $R^3$ est H ou $C_2H_5$ et n est égal à 2 ou 3.

2. Un composé selon la revendication 1, dans lequel $R^1$ est un groupe trityle.

3. Un composé selon la revendication 1, qui est l'acide (Z)-2-(2-tritylaminothiazole-4-yl)-2-[(2-pyrrolidone-3-yl)-oxyimino]acétique.

4. Un composé selon la revendication 1, qui est le(Z)-2-(2-tritylaminothiazole-4-yl)-2-[(2-pyrrolidone-3-yl)oxyimino]acétate d'éthyle.

5. Un composé selon la revendication 1, qui est l'acide (Z)-2-(2-tritylaminothiazole-4-yl)-2-[(1-méthyl-2-pyrrolidone-3-yl) oxyimino]acétique.

6. Un composé selon la revendication 1, qui est le (Z)-2-(2-tritylaminothiazole-4-yl)-2-[(1-méthyl-2-pyrrolidone-3-yl)oxyimino]acétate d'éthyle.

7. Un composé selon la revendication 1, qui est l'acide (Z)-2-(2-tritylaminothiazole-4-yl)-2-[(2-pipéridone-3-yl)oxyimino]acétique.

8. Un composé selon la revendication 1, qui est l'acide (Z)-2-(2-tritylaminothiazole-4-yl)-2-[((3S)-2-pyrrolidone-3-yl)oxyimino]acétique.

9. Un composé selon la revendication 1, qui est l'acide (Z)-2-(2-tritylaminothiazole-4-yl)-2-[((3R)-2-pyrrolidone-3-yl)oxyimino]acétique.

10. Un procédé pour préparer un composé répondant à la formule indiquée dans la revendication 1, qui consiste à faire réagir un composé de formule:

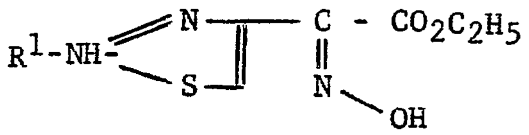

(II)

dans laquelle $R^1$ a la signification indiquée dans la revendication 1, avec un composé de formule:

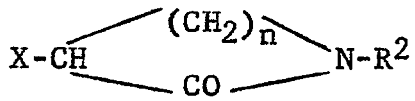

(III)

dans laquelle X est un halogène et $R^2$ et n sont définis comme dans la revendication 1 pour former un composé de formule:

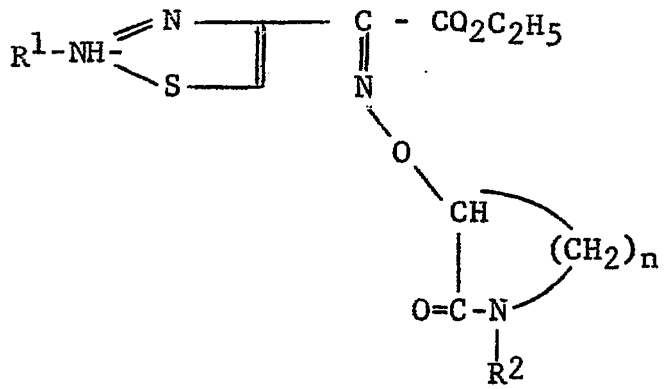

(IV)

dans laquelle $R^1$, et $R^2$ ont la signification indiquée dans la revendication, et, si on le désire, à hydrolyser le composé de formule IV.

11. Un procédé pour préparer un composé de formule:

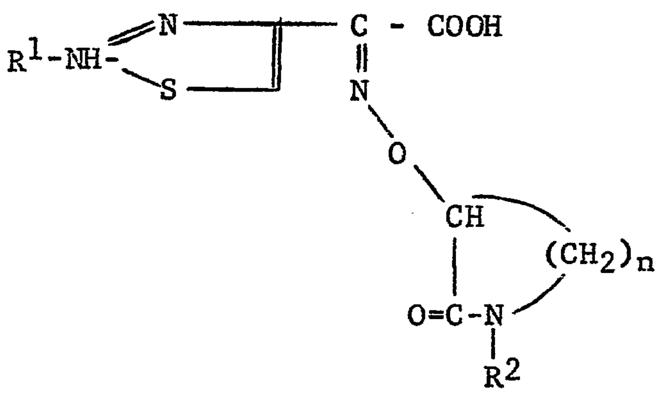

(VI)

dans laquelle $R^1$, $R^2$ et n ont les significations indiquées à la revendication 1, qui consiste à hydrolyser un composé de formule

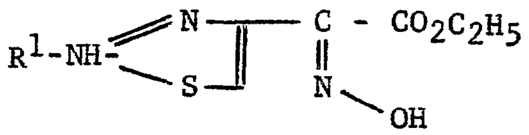

(II)

dans laquelle $R^1$ est défini dans la revendication 1 pour donner un composé de formule:

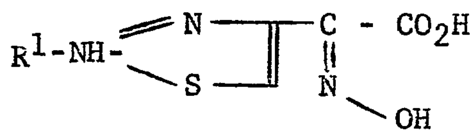

(V)

dans laquelle $R^1$ a la signification indiquée dans la revendication 1 et ensuite à faire réagir le composé V avec un composé de formule:

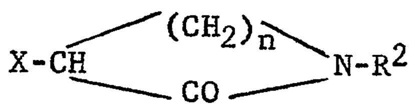

(III)

12. Un procédé pour préparer un composé optiquement actif de formule:

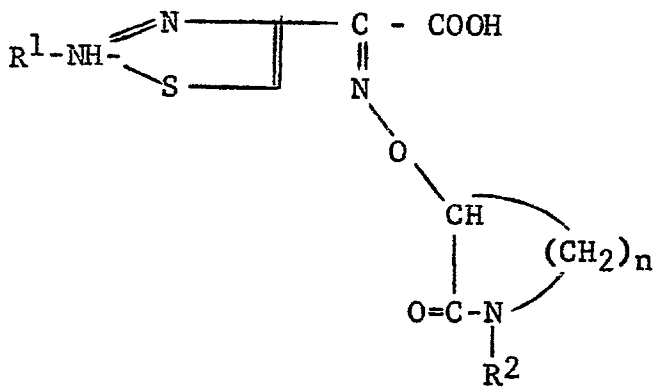

(VI)

dans laquelle $R^1$, $R^2$ et n ont les significations indiquées dans la revendication 1, caractérisé en ce qu'on fait réagir une forme racémique d'un composé de formule VI avec un agent de résolution optiquement actif pour former 2 de ses sels diastéréoisomères et on sépare lesdits sels diastéréoisomères l'un de l'autre.

13. Un procédé selon la revendication 12, caractérisé en ce que l'agent de résolution optiquement actif est l'ester de méthyle de L- ou D-phénylalanine et la séparation des diastéréoisomères est effectuée par recristallisation sélective.

14. Un procédé selon la revendication 13, caractérisé en ce que la recristallisation sélective est effectuée à une température de 10 à 40°C.

**Patentansprüche**

1. Verbindung, gekennzeichnet durch die Formel

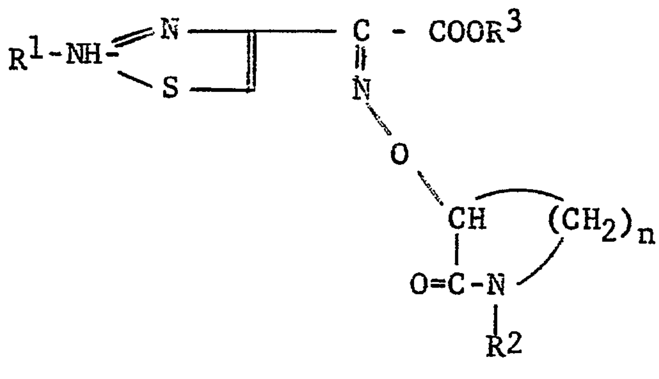

(I)

worin $R^1$ eine Schutzgruppe, die üblicherweise zum Schutz von Aminogruppen bei der Peptidsynthese verwendet wird, bedeutet, $R^2$ für H oder einen Alkylrest mit 1 bis 4 Kohlenstoffatomen steht, $R^3$ H oder $C_2H_5$ darstellt und n eine ganze Zahl, 2 oder 3, ist.

2. Verbindung, wie in Anspruch 1 beansprucht, worin $R^1$ eine Tritylgruppe bedeutet.

3. Verbindung, wie in Anspruch 1 beansprucht, welche (Z)-2-(2-Tritylaminothiazol-4-yl)-2-[(2-pyrrolidon-3-yl)-oxyimino]-essigsäure ist.

4. Verbindung, wie in Anspruch 1 beansprucht, welche Äthyl-(Z)-2-(2-Tritylaminothiazol-4-yl)-2-[(2-pyrrolidon-3-yl)-oxyimino]-acetat ist.

5. Verbindung, wie in Anspruch 1 beansprucht, welche (Z)-2-(2-Tritylaminothiazol-4-yl)-2-[(1-methyl-2-pyrrolidon-3-yl)-oxyimino]-essigsäure ist.

6. Verbindung, wie in Anspruch 1 beansprucht, welche Äthyl-(Z)-2-(2-tritylaminothiazol-4-yl)-2-[(-methyl-2-pyrrolidon-3-yl)-oxyimino]-acetat ist.

7. Verbindung, wie in Anspruch 1 beansprucht, welche (Z)-2-(2-Tritylaminothiazol-4-yl)-2-[(2-piperidon-3-yl)-oxyimino]-essigsäure ist.

8. Verbindung, wie in Anspruch 1 beansprucht, welche (Z)-2-(2-Tritylaminothiazo-4-yl)-2-[((3S)-2-pzrrolidon-3-yl)-oxyimino]-essigsäure ist.

9. Verbindung, wie in Anspruch 1 beansprucht, welche (Z)-2-(2-Tritylaminothiazo-4-yl)-2-[((3R)-2-pyrrolidon-3-yl)-oxyimino]-essigsäure ist.

10. Verfahren zur Herstellung einer Verbindung der in Anspruch 1 angegebenen Formel, welches die Umsetzung einer Verbindung der Formel

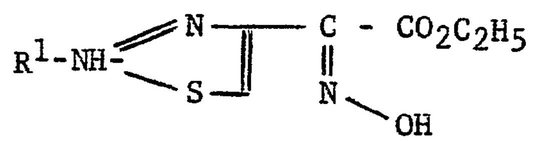

(II)

worin $R^1$ die in Anspruch 1 angegebene Bedeutung hat, mit einer Verbindung der Formel

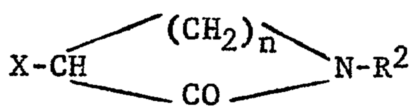

(III)

worin X Halogen bedeutet und $R^2$ und n die in Anspruch 1 angegebene Bedeutung haben, zur Bildung einer Verbindung der Formel

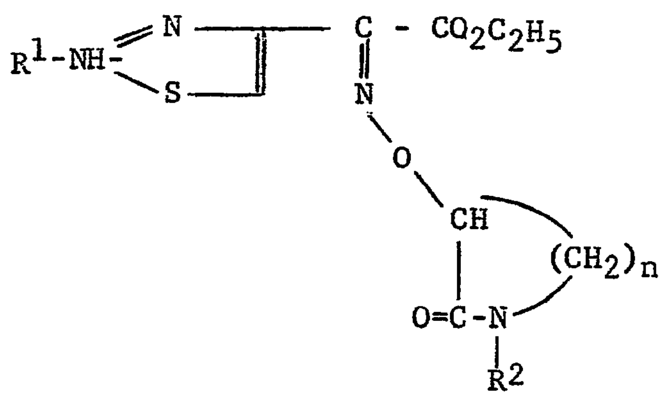

(IV)

worin $R^1$ und $R^2$ die in Anspruch 1 angegebene Bedeutung haben, und gewünschtenfalls Hydrolyse der Verbindung der Formel IV umfaßt.

11. Verfahren zur Herstellung einer Verbindung der Formel

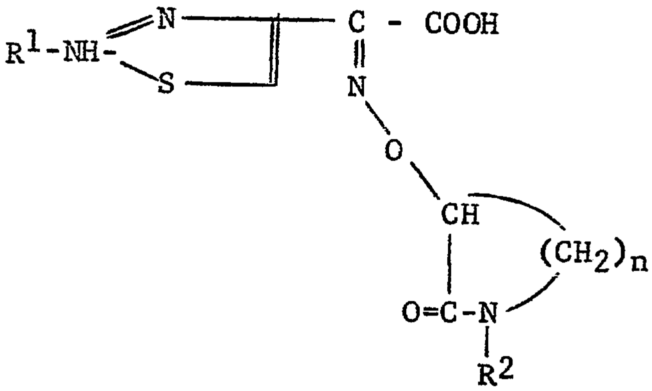

(VI)

worin $R^1$, $R^2$ und n die in Anspruch 1 angegebene Bedeutung haben, welches die Hydrolyse einer Verbindung der Formel:

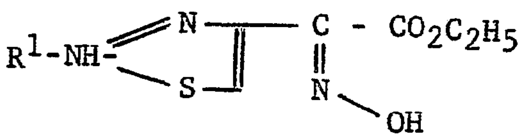

(II)

worin $R^1$ die in Anspruch 1 angegebene Bedeutung hat, zum Erhalt einer Verbindung der Formel

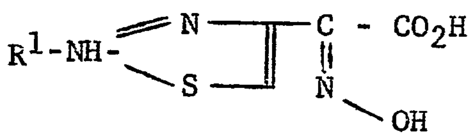

(V)

worin $R^1$ die in Anspruch 1 angegebene Bedeutung hat, und nachfolgende Umsetzung der Verbindung V mit einer Verbindung der Formel

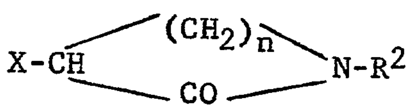

(III)

umfaßt.

12. Verfahren zur Herstellung einer optisch aktiven Verbindung der Formel

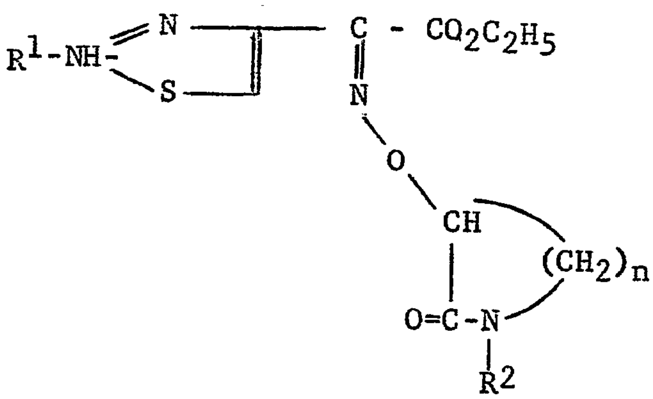

(IV)

worin $R^1$ und $R^2$ und n die in Anspruch 1 angegebene Bedeutung haben, gekennzeichnet durch die Schritte der Umsetzung einer racemischen Form einer Verbindung der Formel VI mit einem optisch aktiven Spaltungsmittel zur Bildung zweier Diastereoisomerensalze davon, der Trennung dieser Diastereoisomerensalze voneinander.

13. Verfahren, wie in Anspruch 12 beansprucht, dadurch gekennzeichnet, daß das optisch aktive Spaltungsmittel ein L- oder D-Phenylalaninmethylester ist und die Trennung der Diastereoisomeren durch selektive Kristallisation bewirkt wird.

14. Verfahren, wie in Anspruch 13 beansprucht, dadurch gekennzeichnet, daß die selektive Kristallisation bei einer Temperatur von 10° bis 40°C bewirkt wird.